# NOUVEAU FASCICULE DE BREVET EUROPEEN

(11) **EP 1 362 572 B2**
(45) Date de publication et mention de la décision concernant l'opposition: **17.08.2011**
(45) Mention de la délivrance du brevet: 16.04.2008
(21) Numéro de dépôt: 03291040.8
(22) Date de dépôt: 29.04.2003
(51) Int. Cl.: A61Q 5/02, A61Q 5/12

(54) **Utilisation d'au moins un sel organique particulier pour le lavage et/ou le conditionnement des matières kératiniques, utilisation d'un sel organique particulier comme agent de conditionnement desdites matières et procédé de traitement cosmétique**
Verwendung eines besonderen organischen Salzes zum Waschen und/oder Konditionieren von Keratinfasern, Verwendung eines besonderen organischen Salzes als Konditionierungsmittel für diese Fasern und ein Verfahren zur kosmetischen Behandlung
Use of at least one particular organic salt for cleansing and/or conditioning keratinous materials, use of a particular organic salt as conditioning agent for said materials and process for cosmetic treatment

(30) Priorité: 14.05.2002 FR 0205913
(43) Date de publication de la demande: 19.11.2003
(73) Titulaire: L'Oréal, 75008 Paris (FR)
(72) Inventeur: Giroud, Franck, 92110 Clichy (FR)
(74) Mandataire: Bourdeau, Françoise

(56) Documents cités:
- EP-A- 1 182 196
- EP-A- 1 182 197
- EP-A2- 0 116 439
- EP-A2- 0 539 251
- DE-A1- 3 833 972
- US-A- 3 989 711
- US-A- 4 732 990
- US-B1- 7 163 786
- N. KIMIZUKA AND T. NAKASHIMA: "Spontaneous Self-Assembly of Glycolipid Bilayer Membranes in Sugar-philic Ionic Liquids and Formation of Ionogels" LANGMUIR, vol. 17, 2001, pages 6759-6761, XP002219946
- M. FREEMANTLE: "New Horizons for Ionic Liquids" CHEMICAL AND ENGINEERING NEWS, 2001, pages 21-25, XP001053911
- WALDEN P: "UEBER DIE MOLEKULARGROESSE UND ELEKTRISCHE LEITFAEHIGKEIT EINIGER GESCHMOLZENEN SALZE" BULLETIN DE L'ACADEMIE IMPERIALE DES SCIENCES DE ST-PETERSBOURG, ACADEMIE IMPERIALE DES SCIENCES, ST-PETERSBOURG, RU, 1914, pages 405-422, XP001135211 ISSN: 1606-6073
- DATABASE WPI Week 200245 Derwent Publications Ltd., London, GB; AN 2002-419661 XP002219947 & JP 2002 003478 A (KAGAKU GIJUTSU SHINKO JIGYODAN), 9 janvier 2002 (2002-01-09)
- PIETRO FALCIOLA: 'Sulla separazione degli acidi grassi liquidi' GAZZETTA CHIMICA ITALIANA vol. 40, no. II, 1911, pages 425 - 435
- CHEMICAL ABSTRACTS, 08 Septembre 1998, Columbus, Ohio, US; abrégé no. 1998:589456, ' Abstract JP 10236930 A'
- OTTO WESTPHAL: 'Über die Umsetzung von höheren 1-Chlor-Paraffinen mit Ammoniak,primären, sekundären und tertiären Aminen' BERICHTE DER DEUTSCHEN CHEMISCHEN GESELLSCHAFT vol. 9, 07 Août 1940, pages 1002 - 1011
- HANS-PETER KRIMMER: 'Ein neues einfaches Verfahren zur Herstellung von Salzen der L-Pyroglutaminsäure' CHEMIKER-ZEITUNG vol. 114, Avril 1990, pages 117 - 121
- FEY OTTE: 'Wörterbuch der Kosmetik', 1997, WISSENSCHAFTLICHE VERL, STUTTGART page 156

## Description

La présente invention est relative à un procédé de traitement cosmétique des matières kératiniques, et en particulier des cheveux, consistant à appliquer sur lesdites matières, à une température comprise entre 10 et 80°C, une composition cosmétique comprenant, dans un milieu cosmétiquement acceptable, au moins un sel organique, le sel organique étant choisi parmi les sels d'imidazolium, de pyrazolium, de pyridinium, de pyrimidinium et de tétraalkylphosphonium et leurs mélanges.

Dans le domaine de la cosmétique, on cherche notamment à améliorer le conditionnement des matières kératiniques, et en particulier des cheveux. Par conditionnement, on entend des propriétés de démêlage facile, de brillance et de douceur au toucher.

Des composés fluides présentant une faible tension superficielle, tels que les silicones, sont généralement utilisés à cet usage. Ils sont bien connus dans la technique pour améliorer en particulier le démêlage des cheveux.

On peut aussi utiliser à cet effet des sels d'ammonium quaternaire à température de fusion élevée tels que le bromure de cétrimonium, sous la forme d'une solution aqueuse ou d'émulsion aqueuse. Ces deux types de composés permettent donc d'obtenir un excellent conditionnement des cheveux, mais leur utilisation s'accompagne d'inconvénients majeurs. En effet, les cheveux deviennent gras, leur touché est chargé et on observe un transfert de matière sur les doigts.

Les documents EP 1182196 et EP 1182197 décrivent un procédé de fabrication de sels organiques liquides à température ambiante.

JP 2002003478 décrit des sels de N-alkyl-N'-alkoxyalkylimidazolium et prévoit leur utilisation dans divers domaines comme les membranes, les électrolytes, les lubrifiants, les cosmétiques, les conducteurs ioniques, les catalyseurs, les biocatalyseurs, les cristaux liquides, le transport des protéines.

Actuellement, il n'existe pas dans ce domaine, d'autres composés lubrifiants liquides permettant d'obtenir un excellent conditionnement des cheveux sans les inconvénients majeurs cités ci-dessus.

La Demanderesse a trouvé de manière surprenante qu'en utilisant une famille particulière de composés, il était possible d'obtenir un très bon conditionnement des cheveux et de palier aux inconvénients des composés cités ci-dessus, utilisés dans les compositions cosmétiques de l'art antérieur.

Cette famille particulière de composés est constituée par des sels organiques non polymériques présentant un point de fusion inférieur à 60 °C, le sel organique étant choisi parmi les sels d'imidazolium, de pyrazolium, de pyridinium, de pyrimidinium et de tétraalkylphosphonium et leurs mélanges.

Ces sels organiques font partie d'une classe générale de composés bien connus sous le nom de "Room Temperature Ionic Liquid ou RTIL". Ces RTIL présentent généralement un point de fusion inférieur à 100 °C et restent liquides jusqu'à une température de 300 °C environ.

Ces RTIL sont notamment décrits dans " Eyes on Ionic Liquids, Chemical and Engineering News", May 15, 2000, Vol. 78, 20, pages 37-50 et dans "New Horizons For Ionic Liquids, Chemical and Engineering News", January 1, 2001, Vol. 79, 1, pages 21-25.

Le point de fusion est mesuré par analyse calorimétrique différentielle (DSC) avec une vitesse de montée en température de 10 °C/min. Le point de fusion est alors la température correspondant au sommet du pic endotherme de fusion obtenu lors de la mesure.

Les sels organiques du procédé de l'invention possèdent en outre un excellent pouvoir de solvatation et une excellente conductivité électrique. Ils sont également non-volatiles et ininflammables.

Un autre avantage de ces sels organiques réside dans le fait qu'ils sont facilement recyclables et font partie des solvants dits "verts".

L'invention a donc pour objet un procédé de traitement cosmétique des matières kératiniques, et en particulier des cheveux, consistant à appliquer sur lesdites matières, à une température comprise entre 10 et 80°C, une composition cosmétique comprenant, dans un milieu cosmétiquement acceptable, au moins un sel organique présentant un point de fusion inférieur à 60°C et à rincer éventuellement après un temps de pose compris entre 15 secondes et 30 minutes, le sel organique étant choisi parmi les sels d'imidazolium, de pyrazolium, de pyridinium, de pyrimidinium et de tétraalkylphosphonium et leurs mélanges.

Un autre objet de la présente invention est une utilisation de la composition cosmétique ci-dessus pour le lavage et/ou le conditionnement matières kératiniques, et en particulier des cheveux, le sel organique étant choisi parmi les sels d'imidazolium, de pyrazolium, de pyridinium, de pyrimidinium et de tétraalkylphosphonium et leurs mélanges.

Elle a encore pour objet l'utilisation d'un sel organique non polymérique présentant un point de fusion inférieur à 60 °C, comme agent de conditionnement des matières kératiniques, et en particulier des cheveux, le sel organique étant choisi parmi les sels d'imidazolium, de pyrazolium, de pyridinium, de pyrimidinium et de tétraalkylphosphonium et leurs mélanges.

D'autres objets, caractéristiques, aspects et avantages de l'invention apparaîtront encore plus clairement à la lecture de la description et des divers exemples qui suivent.

La composition cosmétique du procédé selon l'invention comprend, dans un milieu cosmétiquement acceptable, au moins un sel organique non polymérique présentant un point de fusion inférieur à 60 °C. Ce point de fusion est de préférence inférieur à 20 °C, mieux encore inférieur à 0°C et encore plus préférentiellement inférieur à -30 °C et le sel organique étant choisi parmi les sels d'imidazolium, de pyrazolium, de pyridinium, de pyrimidinium et de tétraalkylphosphonium et leurs mélanges.

Par milieu cosmétiquement acceptable, on entend un milieu compatible avec toutes les matières kératiniques telles que la peau, les cheveux, les ongles, les cils et sourcils, les lèvres et toute autre zone du corps et du visage.

Les sels organiques utilisés dans la composition cosmétique du procédé selon l'invention, sont choisis parmi les sels d'imidazolium, de pyrazolium, de pyridinium, de pyrimidinium, et de tétra-alkylphosphonium de point de fusion inférieur à 60 °C, et leurs mélanges.

A titre d'exemples de sels d'imidazolium, on peut notamment citer ceux de formule (I) : 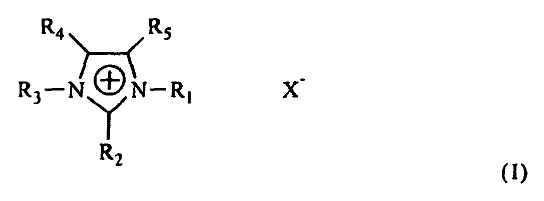 dans laquelle :
R₁ et R₃, identiques ou différents, représentent chacun un groupe alkyle comportant de 1 à 12 atomes de carbone, de préférence de 1 à 5 atomes de carbone et mieux encore de 1 à 4 atomes de carbone,
R₂, R₄ et R₅, identiques ou différents, représentent chacun un atome d'hydrogène ou un groupe alkyle comportant de 1 à 4 atomes de carbone et de préférence de 1 à 3 atomes de carbone, et
X⁻ représente un anion.

Les groupes alkyles peuvent être linéaires ou ramifiés. On peut notamment citer, par exemple, les groupes méthyle, éthyle, n-propyle, isopropyle, n-butyle, tert-butyle, pentyle, hexyle, octyle, nonyle, décyle et dodécyle.

On utilise de préférence dans la composition du procédé de l'invention, les sels d'alkyl-méthylimidazolium, et plus spécifiquement les sels de 1-butyl-3-méthylimidazolium ou de 1-éthyl-3-méthylimidazolium.

Comme sels de pyrazolium utilisables dans la présente invention, on peut notamment citer ceux de formule (II) : 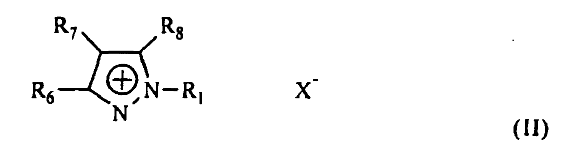 dans laquelle :
R₁ et X⁻ a la même signification que ci-dessus, et
R₆, R₇ et R₈, identiques ou différents, représentent chacun un atome d'hydrogène ou un groupe alkyle comportant de 1 à 5 atomes de carbone et de préférence de 1 à 4 atomes de carbone.

Comme exemples de sels de pyridinium pouvant être utilisés dans la composition du procédé de l'invention, on peut notamment citer ceux de formule (III) : 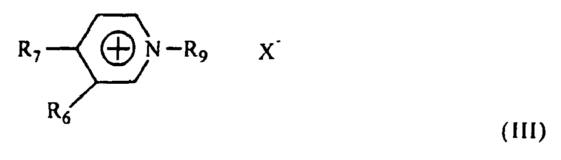 dans laquelle R₆, R₇, et X⁻ sont tels que définis ci-dessus, et R₉ représente un groupe alkyle comportant de 1 à 8 atomes de carbone, et de préférence de 1 à 4 atomes de carbone. et plus particulièrement les sels de N-butylpyrdinium.

Comme sels de pyrimidinium utilisables dans la présente invention, on peut notamment citer ceux de formules (IV) et (IV') : 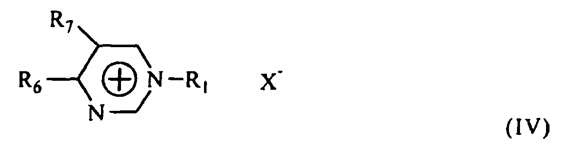 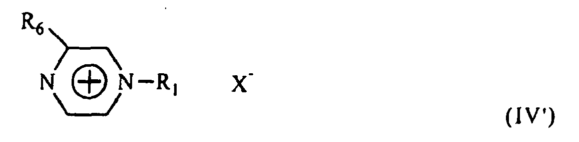 dans lesquelles R₁, R₆, R₇, et X⁻ sont tels que définis ci-dessus.

D'autres sels organiques existent sous la forme de cations non hétérocycliques tels que les sels de tétra-alkylphosphonium de point de fusion inférieur à 60 °C.

Comme sels de tétra-alkylphosphonium utilisables dans la présente invention, on peut notamment citer ceux de formule (V) : 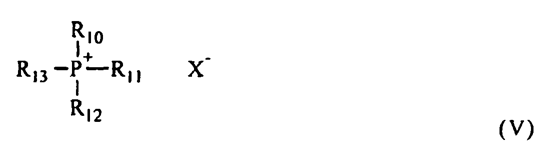 dans laquelle R₁₀, R₁₁, R₁₂ et R₁₃, identiques ou différents, représentent un groupe alkyle comportant de 1 à 18 atomes de carbone et de préférence de 1 à 14 atomes de carbone, et X⁻ représente un anion.

L'anion présent dans le sel organique, représenté par X⁻, peut être tout anion bien connu dans la technique, et en particulier un ion chlorure (Cl⁻), un ion bromure (Br⁻), un ion tétrachloroaluminate (AlCl₄⁻), un ion tétrachloronickel (NiC1₄⁻), un ion perchlorate (ClO₄⁻), , un ion nitrate (NO₃⁻), un ion nitrite (NO₂⁻), un ion sulfate (SO₄²⁻), un ion méthylsulfate (CH₃SO₄⁻), un ion tétrafluoroborate (BF₄⁻), un ion hexafluorophosphate (PF₆⁻), un ion hexafluoroantimonate (SbF₆⁻), un ion triflate [TfO] (CF₃SO₂⁻) un ion nonaflate [NfO] (CF₃(CF₂)₃SO₂⁻), un ion bis(trifyl)amide [Tf₂N] (CF₃SO₂)₂N⁻), un ion trifluoroacétate [TA] (CF₃CO₂⁻), un ion heptafluorobutanoate [HB] (CF₃(CF₂)₃CO₂⁻), un ion acétate (CH₃CO₂⁻), un ion trifluoroacétate (CF₃CO₂⁻), ou un ion trifluorométhanesulfonate (CF₃SO₃⁻).

Parmi les anions cités ci-dessus, on préfère tout particulièrement les ions chlorure, bromure, sulfate, acétate, tétrafluoroborate, hexafluorophosphate, triflate, nonaflate, bis(trifyl)amide et heptafluorobutanoate.

A titre d'exemples de sel organique utilisés dans le cadre de la présente invention, on peut notamment citer les sels organiques suivants :
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de 1-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le tétrafluoroborate de 1-éthyl-3-méthylimidazolium,
le tétrafluoroborate de 1-butyl-3-méthylimidazolium,
le tétrafluoroborate de 1-pentyl-3-méthylimidazolium,
le tétrafluoroborate de 1-hexyl-3-méthylimidazolium,
le tétrafluoroborate de 1-heptyl-3-méthylimidazolium,
le tétrafluoroborate de 1-octyl-3-méthylimidazolium,
le tétrafluoroborate de 1-nonyl-3-méthylimidazolium,
le tétrafluoroborate de 1-décyl-3-méthylimidazolium,
le tétrafluoroborate de 4-méthyl-N-butylpyridinium,
le tétrafluoroborate de 1-hexyl-3-éthylimidazolium,
l'hexafluorophosphate de 1-éthyl-3-méthylimidazolium,
l'hexafluorophosphate de 1-butyl-3-méthylimidazolium,
l'hexafluorophosphate de 1-pentyl-3-méthylimidazolium,
l'hexafluorophosphate de 1-hexyl-3-méthylimidazolium,
l'hexafluorophosphate de 1-heptyl-3-méthylimidazolium,
l'hexafluorophosphate de 1-octyl-3-méthylimidazolium,
l'hexafluorophosphate de 1-nonyl-3-méthylimidazolium,
l'hexafluorophosphate de 1-décyl-3-méthylimidazolium,
le méthylsulfate de 1,3-diméthylimidazolium,
le méthylsulfate de 1-méthyl-3-butylimidazolium,
le nitrate de 1-éthyl-3-méthylimidazolium,
le nitrite de 1-éthyl-3-méthylimidazolium,
l'acétate de 1-éthyl-3-méthylimidazolium,
le sulfate de 1-éthyl-3-méthylimidazolium,
les triflates de 1-éthyl-3méthylimidazolium,
les nonaflates de l-éthyl-3-méthylimidazolium,
le bis(trifyl)amide de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le chlorure de trihexyl-tétradécyl-phosphonium,
le chlorure de tributyl-tétradécyl-phosphonium,
le tétrafluoroborate de 1-éthyl-2-méthylpyrazolium, et
le tétrafluoroborate de 1-méthyl-3-butylpyrimidinium.

Il est possible de modifier la chimie des sels organiques telle qu'elle a été décrite précédemment pour faire varier leur solubilité. Pour conserver le caractère fusible à basse température de ces sels, il est préférable de modifier plus spécifiquement les chaînes alkyle constituant en partie les sels organiques. Dans ce cadre, on peut citer, à titre d'exemple, l'éthérification de la chaîne alkyle des sels de la famille des 1-alkyl-3-méthylimidazolium qui permet d'obtenir les composés de formules suivantes :

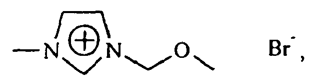

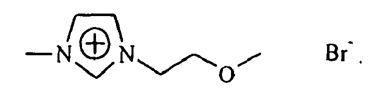

Une telle modification chimique donne ou renforce pour le sel organique le caractère hydrosoluble, sans perdre le caractère fusible à basse température du sel. Une telle modification chimique peut également permettre de faire gélifier les sels organiques par association avec des sucres amphiphiles et d'obtenir ce que l'on appelle des ionogels. De telles propriétés sont décrites dans l'article "Spontaneous Self assembly of glycolipid bilayer Membranes in Sugar-philic Ionic Liquids and formation of Ionogels" publié par N. Kimizuka et T Nakashima (Langmuir 17, 6759-6761, (2001)).

Le sel organique ou le mélange de sels organiques tels que décrits ci-dessus, est présent dans les compositions cosmétiques du procédé de l'invention, en une concentration comprise entre 0,00001% et 99,9999% en poids, de préférence entre 0,01% et 50% en poids, et mieux encore entre 0,1% et 30% en poids, par rapport au poids total de la composition.

Le sel organique ou le mélange de sels organiques peut se présenter en solution ou sous forme d'une émulsion. Le sel organique ou le mélange de sels organiques peut être également préalablement microencapsulé avant incorporation dans la composition cosmétique.

Le sel organique tel que décrit ci-dessus est notamment utilisé en cosmétique comme agent de conditionnement.

Le milieu cosmétiquement acceptable de la composition cosmétique du procédé de l'invention peut contenir un composé ou un mélange de composés cosmétiquement acceptables permettant de véhiculer les sels organiques. Ce véhicule peut être constitué par l'un des composés suivants ou un mélange des composés suivants : l'eau ; les alcools aliphatiques en C₁-C₄ et plus particulièrement l'éthanol ; les alcools aromatiques tels que l'alcool benzylique ; les alcools gras, saturés ou insaturés, en C₁₀-C₃₀ ; les polyols modifiés ou non tels que le glycérol, le propylèneglycol, le dipropylèneglycol, le butylèneglycol, le butyle diglycol, les polyéthylèneglycols ; les silicones volatiles ou non telles que le cylopentasiloxane, les polydiméthylsiloxanes modifiés ou non par des fonctions phényle et/ou siloxy et/ou silanol et/ou amine et/ou imine et/ou fluoroalkyle et/ou carboxylique et/ou betaïne et/ou ammonium quaternaire ; les huiles minérales, organiques ou végétales ; les cires oxyéthylénées ou non, les paraffines ; les alcanes et plus particulièrement les alcanes en C₅-C₁₀ ; les acides gras en C₁₀-C₃₀ ; les amides grasses en C₁₀-C₃₀ , les esters gras en C₁₀-C₃₀ et plus particulièrement les benzoates ou les salicylates d'alcool gras.

Le milieu cosmétiquement acceptable peut également contenir au moins un autre composé tel que l'acétone, la méthyléthylcétone, l'acétate de méthyle, l'acétate de butyle, l'acétate d'éthyle, le diméthoxyéthane et le diéthoxyéthane.

La composition du procédé selon l'invention peut également contenir un agent propulseur. A titre d'agents propulseurs, on peut notamment citer les gaz comprimés ou liquéfiés généralement utilisés pour la préparation de compositions aérosols. On peut notamment citer comme exemple d'agent propulseur, l'air, le gaz carbonique, l'azote comprimé ou encore un gaz soluble tel que le diméthyléther, les hydrocarbures halogénés, par exemple, fluorés, ou les hydrocarbures non halogénés, et leurs mélanges.

Les compositions du procédé de l'invention peuvent contenir en outre au moins un additif cosmétique couramment utilisé dans la technique tel que, par exemple, les agents réducteurs, les agents oxydants, les corps gras, les silicones, les agents épaississants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les parfums, les peptisants, les conservateurs, les tensioactifs anioniques, amphotères, zwitterioniques ou non-ioniques, les polymères fixants ou non, les polymères conditionneurs, les protéines et les vitamines.

L'homme de métier veillera à choisir les éventuels additifs cosmétiques et leur quantité de manière à ce qu'ils ne nuisent pas aux propriétés des compositions du procédé de la présente invention.

Ces adjuvants sont généralement présents dans la composition du procédé selon l'invention en une quantité allant de 0 à 20 % en poids par rapport au poids total de la composition.

Les compositions cosmétiques du procédé de l'invention peuvent se présenter sous différentes formes galéniques telles qu'une lotion, un spray, une mousse, un après-shampoing ou un shampoing.

Lorsque la composition est utilisée pour le lavage et/ou le conditionnement des matières kératiniques, notamment des cheveux, elle peut être utilisée en mode rincé ou non.

Les exemples suivants sont donnés à titre illustratif de la présente invention.

### Exemple 1 et exemple comparatif 1 : compositions rincées

L'exemple 1 concerne un shampoing utilisé dans le procédé selon l'invention tandis que l'exemple comparatif 1 concerne un shampoing témoin.

On prépare les shampoings à partir des ingrédients suivants dans les proportions suivantes indiquées en % en poids.

| | Ex. 1 | Ex. comp. 1 |
|---|---|---|
| Laurethsulfate de sodium⁽¹⁾ | 30 MA | 30 MA |
| Coco-bétaïne ⁽²⁾ | 4 MA | 4 MA |
| Cocamide MIPA ⁽³⁾ | 2 MA | 2 MA |
| PDMS (PM 250 000)⁽⁴⁾ | - | 1,5 MA |
| Tétrafluoroborate de 1-méthyl-3-butylimidazolium ⁽⁵⁾ | 1,5 MA | - |
| Cétéarylsulfate de sodium⁽⁶⁾ | 0,8 MA | 0,8 MA |
| Polyquaternium-10⁽⁷⁾ | 0,4 MA | 0,4 MA |
| Carbomer ⁽⁸⁾ | 0,2 MA | 0,2 MA |
| Propylèneglycol | 0,1 MA | 0,1 MA |
| Conservateur | qs | qs |
| Parfum | qs | qs |
| Eau qsp | 100 | 100 |

| | | |
|---|---|---|
| M.A. : matière active ⁽¹⁾ TEXAPON N 702 commercialisé par la société COGNIS, ⁽²⁾ DEHYTON AB 30 OR commercialisé par la société COGNIS, ⁽³⁾ EMPILAN CIS commercialisé par la société HUNSTMAN, ⁽⁴⁾ MIRASIL DM 500 000 commercialisé par la société RHODIA, ⁽⁵⁾ commercialisé par la société SOLVENT INNOVATION Gmbh sous la référence 99,020-1, ⁽⁶⁾ LANETTE E commercialisée par la société COGNIS, ⁽⁷⁾ UCARE POLYMER JR 400 commercialisé par la société AMERCHOL, ⁽⁸⁾ CARBOPOL 980 commercialisé par la société NOVEON. | | |

On a ensuite soumis ces deux formulations de shampoing à une évaluation des caractéristiques cosmétiques conférées aux cheveux.

On a appliqué 5 g de shampoing sur une chevelure constituée par des cheveux caucasiens naturels châtains de 20 cm de long. Au bout de 2 minutes de pose, la chevelure a été rincée à l'eau, puis coiffée à l'aide d'un sèche-cheveux.

Cet essai a été réalisé sur 20 modèles : 10 modèles avec le shampoing de l'exemple 1 et les 10 autres avec le shampoing de l'exemple comparatif 1.

Les caractéristiques cosmétiques des chevelures ont ensuite été évaluées par un panel comprenant 10 experts.

Ce panel a indiqué que toutes les chevelures traitées avec le shampoing de l'exemple 1, c'est-à-dire dans le procédé de l'invention, sont plus douces, plus lisses, moins grasses et moins chargées au toucher, que les chevelures traitées avec le shampoing de l'exemple comparatif 1.

### Exemple 2 et exemple comparatif 2 : compositions non-rincées

L'exemple 2 concerne une crème de brillance utilisée dans le procédé selon l'invention tandis que l'exemple comparatif 2 concerne une crème de brillance témoin.

On prépare les crèmes de brillance à partir des ingrédients suivants dans les proportions suivantes indiquées en % en poids.

| | Ex. 2 | Ex. comp. 2 |
|---|---|---|
| Cyclopentasiloxane ⁽¹⁾ | - | 10 MA |
| Tétrafluoroborate de 1-méthyl-3-butylimidazolium ⁽²⁾ | 10 MA | - |
| Cyclopentasiloxane diméthicone copolyol ⁽³⁾ | 0,5 MA | 0,5 MA |
| Propylèneglycol | 2,5 MA | 2,5 MA |
| Conservateur | qs | qs |
| Parfum | qs | qs |
| Eau qsp | 100 | 100 |

| | | |
|---|---|---|
| M.A. : matière active ⁽¹⁾ MIRASIL CM 5 commercialisé par la société RHODIA, ⁽²⁾ commercialisé par la société SOLVENT INNOVATION Gmbh sous la référence 99,020-1, ⁽³⁾ DOW CORNING 5225C commercialisé par la société DOW CORNING, | | |

On a ensuite soumis ces deux formulations de crème de brillance à une évaluation des caractéristiques cosmétiques conférées aux cheveux.

On a appliqué 5 g de crème de brillance sur une chevelure constituée par des cheveux caucasiens naturels châtains de 20 cm de long.

Cet essai a été réalisé sur 20 modèles : 10 modèles avec la crème de brillance de l'exemple 2 et les 10 autres avec la crème de brillance de l'exemple comparatif 2.

Les caractéristiques cosmétiques des chevelures ont ensuite été évaluées par un panel comprenant 10 experts, au bout de 10 minutes de pose.

Ce panel a indiqué que toutes les chevelures traitées avec la crème de brillance de l'exemple 2, c'est-à-dire dans le procédé de l'invention, sont moins grasses et moins chargées au toucher que les chevelures traitées avec la crème de brillance de l'exemple comparatif 2. En outre, les chevelures traitées avec la crème de brillance de l'exemple 2, sont plus brillantes.

## Revendications

1. Procédé de traitement cosmétique des matières kératiniques, et en particulier des cheveux, consistant à appliquer sur lesdites matières, à une température comprise entre 10 et 80°C, une composition cosmétique comprenant, dans un milieu cosmétiquement acceptable, au moins un sel organique présentant un point de fusion inférieur à 60°C et à rincer éventuellement après un temps de pose compris entre 15 secondes et 30 minutes, le sel organique étant choisi parmi les sels d'imidazolium, de pyrazolium, de pyridinium, de pyrimidinium et de tétra-alkylphosphonium, et leurs mélanges.

2. Procédé selon la revendication 1, **caractérisé en ce que** les sels d'imidazolium répondent à la formule suivante : 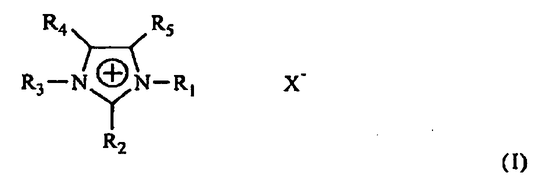 dans laquelle R₁ et R₃, identiques ou différents, représentent chacun un groupe alkyle comportant de 1 à 12 atomes de carbone,
R₂, R₄ et R₅, identiques ou différents, représentent chacun un atome d'hydrogène ou un groupe alkyle comportant de 1 à 4 atomes de carbone, et
X⁻ représente un anion.

3. Procédé selon la revendication 2, **caractérisé en ce que** R₁ et R₃, identiques ou différents, représentent chacun un groupe alkyle comportant de 1 à 5 atomes de carbone,

4. Procédé selon la revendication 1, **caractérisé en ce que** les sels de pyrazolium répondent à la formule suivante : 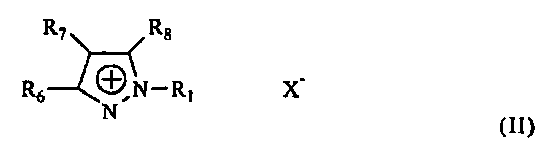 dans laquelle R₁ représente un groupe alkyle comportant de 1 à 18 atomes de carbone, de préférence de 1 à 12 atomes de carbone,
R₆, R₇ et R₈, identiques ou différents, représentent chacun un atome d'hydrogène ou un groupe alkyle comportant de 1 à 5 atomes de carbone et de préférence de 1 à 4 atomes de carbone, et
X⁻ représente un anion.

5. Procédé selon la revendication 1, **caractérisé en ce que** les sels de pyridinium répondent à la formule suivante : 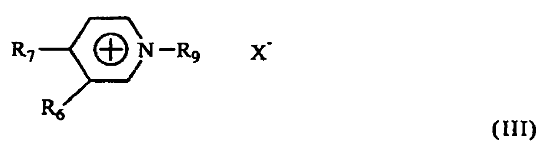 dans laquelle R₉ représente un groupe alkyle comportant de 1 à 8 atomes de carbone, de préférence de 1 à 4 atomes de carbone,
R₆ et R₇, identiques ou différents, représentent chacun un atome d'hydrogène ou un groupe alkyle comportant de 1 à 5 atomes de carbone et de préférence de 1 à 4 atomes de carbone, et
X⁻ représente un anion.

6. Procédé selon la revendication 1, **caractérisé en ce que** les sels de pyrimidinium répondent aux formules suivantes : 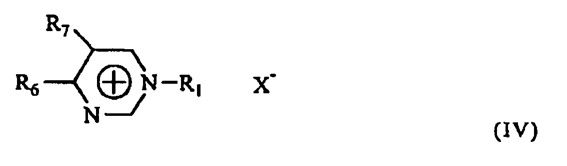 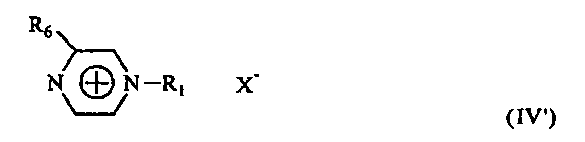 dans lesquelles R₁ représente un groupe alkyle comportant de 1 à 18 atomes de carbone, de préférence de 1 à 12 atomes de carbone,
R₆ et R₇, identiques ou différents, représentent chacun un atome d'hydrogène ou un groupe alkyle comportant de 1 à 5 atomes de carbone et de préférence de 1 à 4 atomes de carbone, et
X⁻ représente un anion.

7. Procédé selon la revendication 1, **caractérisé en ce que** les sels de tétra-alkylphosphonium répondent à la formule suivante : 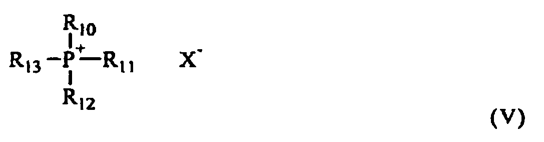 dans laquelle R₁₀, R₁₁ et R₁₂ et R₁₃, identiques ou différents, représentent un groupe alkyle comportant de 1 à 18 atomes de carbone et de préférence de 1 à 14 atomes de carbone, et
X⁻ représente un anion.

8. Procédé cosmétique selon l'une quelconque des revendications précédentes, **caractérisé en ce que** les anions sont choisis parmi les ions chlorure (Cl⁻), bromure (Br⁻), tétrachloroaluminate (AlCl₄⁻), tétrachloronickel (NiCl₄⁻), perchlorate (ClO₄⁻), , nitrate (NO₃⁻), nitrite (NO₂⁻), sulfate (SO₄²⁻), méthylsulfate (CH₃SO₄⁻), tétrafluoroborate (BF₄⁻), hexafluorophosphate (PF₆⁻), hexafluoroantimonate (SbF₆⁻), triflate [TfO] (CF₃SO₂⁻), nonaflate [NfO] (CF₃(CF₂)₃SO₂⁻), un ion bis(trifyl)amide [Tf₂N] (CF₃SO₂)₂N⁻), trifluoroacétate [TA] (CF₃CO₂⁻), heptafluorobutanoate [HB] (CF₃(CF₂)₃CO₂⁻), acétate (CH₃CO₂⁻), trifluoroacétate (CF₃CO₂⁻) et trifluorométhanesulfonate (CF₃SO₃⁻).

9. Procédé cosmétique selon la revendication 1, **caractérisé en ce que** le sel organique est choisi parmi :
le bromure de 1-éthyl-3-méthylimidazolium,
le chlorure de 1-butyl-3-méthylimidazolium,
le chlorure de 1-hexyl-3-méthylimidazolium,
le chlorure de 1-méthyl-3-octylimidazolium,
le chlorure de 1-décyl-3-méthylimidazolium,
le bromure de 1-décyl-3-méthylimidazolium,
le chlorure de 1-dodécyl-3-méthylimidazolium,
le chlorure de l-méthyl-3-tétradécylimidazolium,
le chlorure de 4-méthyl-N-butyl-pyridinium,
le chlorure de 3-méthyl-N-butylpyridinium,
le chlorure de 4-méthyl-N-hexylpyridinium,
le tétrafluoroborate de 1-éthyl-3-méthylimidazolium,
le tétrafluoroborate de 1-butyl-3-méthylimidazolium,
le tétrafluoroborate de 1-pentyl-3-méthylimidazolium,
le tétrafluoroborate de 1-hexyl-3-méthylimidazolium,
le tétrafluoroborate de 1-heptyl-3-méthylimidazolium,
le tétrafluoroborate de 1-octyl-3-méthylimidazolium,
le tétrafluoroborate de 1-nonyl-3-méthylimidazolium,
le tétrafluoroborate de 1-décyl-3-méthylimidazolium,
le tétrafluoroborate de 4-méthyl-N-butylpyridinium,
le tétrafluoroborate de 1-hexyl-3-éthylimidazolium,
l'hexafluorophosphate de 1-éthyl-3-méthylimidazolium,
l'hexafluorophosphate de 1-butyl-3-méthylimidazolium,
l'hexafluorophosphate de 1-pentyl-3-méthylimidazolium,
l'hexafluorophosphate de 1-hexyl-3-méthylimidazolium,
l'hexafluorophosphate de 1-heptyl-3-méthylimidazolium,
l'hexafluorophosphate de 1-octyl-3-méthylimidazolium,
l'hexafluorophosphate de 1-nonyl-3-méthylimidazolium,
l'hexafluorophosphate de 1-décyl-3-méthylimidazolium,
le méthylsulfate de 1,3-diméthylimidazolium,
le méthylsulfate de 1-méthyl-3-butylimidazolium,
le nitrate de 1-éthyl-3-méthylimidazolium,
le nitrite de 1-éthyl-3-méthylimidazolium,
l'acétate de 1-éthyl-3-méthylimidazolium,
le sulfate de 1-éthyl-3-méthylimidazolium,
les triflates de 1-éthyl-3méthylimidazolium,
les nonaflates de 1-éthyl-3-méthylimidazolium,
le bis(trifyl)amide de 1-éthyl-3-méthylimidazolium,
le bromure de 1-butylpyridinium,
le trifluorométhanesulfonate de 1-butylpyrimidinium,
le trifluorométhanesulfonate de 1-hexylpyrimidinium,
le chlorure de trihexyl-tétradécyl-phosphonium,
le chlorure de tributyl-tétradécyl-phosphonium,
le tétrafluoroborate de 1-éthyl-2-méthylpyrazolium,
le tétrafluoroborate de 1-méthyl-3-butylpyrimidinium, et
le trifluoroacétate de 1-éthyl-3-méthylimidazolium.

10. Procédé cosmétique selon l'une quelconque des revendications précédentes, **caractérisé en ce que** le sel organique est contenu en une quantité comprise entre 0,00001% et 99,9999%, de préférence entre 0,01% et 50% en poids par rapport au poids total de la composition.

11. Procédé cosmétique selon l'une quelconque des revendications précédentes, **caractérisé en ce que** le milieu cosmétiquement acceptable comprend un composé cosmétiquement acceptable ou un mélange de composés cosmétiquement acceptables permettant de véhiculer les sels organiques.

12. Procédé selon la revendication 11, **caractérisé en ce que** le milieu cosmétiquement acceptable est choisi parmi l'eau ; les alcools aliphatiques en C₁-C₄ ; les alcools aromatiques ; les alcools gras ; les polyols modifiés ou non ; les silicones volatiles ou non ; les huiles minérales, organiques ou végétales ; les cires oxyéthylénées ou non, les paraffines ; les alcanes en C₅-C₁₀ ; les acides gras ; les amides grasses, les esters gras.

13. procédé cosmétique selon l'une quelconque des revendications précédentes, **caractérisé en ce que** la composition comprend également au moins un composé organique choisi parmi l'acétone, la méthyléthylcétone, l'acétate de méthyle, l'acétate de butyle, l'acétate d'éthyle, le diméthoxyéthane et le diéthoxyéthane.

14. Procédé cosmétique selon l'une quelconque des revendications précédentes, **caractérisé en ce que** la composition comprend en outre au moins un additif cosmétique choisi parmi les agents réducteurs, les agents oxydants, les corps gras, les silicones, les agents épaississants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les parfums, les peptisants, les conservateurs, les tensioactifs anioniques, amphotères, zwitterioniques ou non-ioniques, les polymères fixants ou non, les polymères conditionneurs, les protéines et les vitamines.

15. Utilisation d'un sel organique tel que défini dans l'une quelconque des revendications 1 à 9, comme agent de conditionnement des matières kératiniques, et en particulier des cheveux.

16. Utilisation de la composition selon l'une quelconque des revendications 1 à 14, pour le lavage et/ou le conditionnement des matières kératiniques, et en particulier des cheveux.

## Claims

1. Method for the cosmetic treatment of keratinous substances and in particular the hair, which consists in applying, to the said substances, at a temperature between 10 and 80°C, a cosmetic composition comprising, in a cosmetically acceptable medium, at least one organic salt exhibiting a melting point of less than 60°C, and in optionally rinsing, after a leave-in time of between 15 seconds and 30 minutes, the organic salt being chosen from imidazolium, pyrazolium, pyridinium, pyrimidinium and tetraalkylphosphonium salts, and their mixtures.

2. Method according to Claim 1, **characterized in that** the imidazolium salts correspond to the following formula: 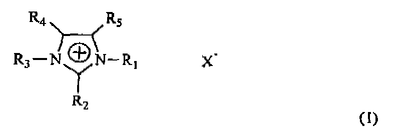
in which R₁ and R₃, which are identical or different, each represent an alkyl group comprising from 1 to 12 carbon atoms,
R₂, R₄ and R₅, which are identical or different, each represent a hydrogen atom or an alkyl group comprising from 1 to 4 carbon atoms, and
X⁻ represents an anion.

3. Method according to Claim 2, **characterized in that** R₁ and R₃, which are identical or different, each represent an alkyl group comprising from 1 to 5 carbon atoms.

4. Method according to Claim 1, **characterized in that** the pyrazolium salts correspond to the following formula: 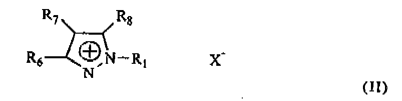
in which R₁ represents an alkyl group comprising from 1 to 18 carbon atoms, preferably from 1 to 12 carbon atoms,
R₆, R₇ and R₆, which are identical or different, each represent a hydrogen atom or an alkyl group comprising from 1 to 5 carbon atoms and preferably from 1 to 4 carbon atoms, and
X⁻ represents an anion.

5. Method according to Claim 1, **characterized in that** the pyridinium salts correspond to the following formula: 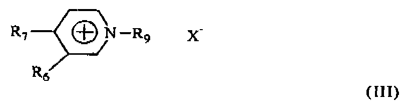
in which R₉ represents an alkyl group comprising from 1 to 8 carbon atoms, preferably from 1 to 4 carbon atoms,
R₆ and R₇, which are identical or different, each represent a hydrogen atom or an alkyl group comprising from 1 to 5 carbon atoms and preferably from 1 to 4 carbon atoms, and
X⁻ represents an anion.

6. Method according to Claim 1, **characterized in that** the pyrimidinium salts correspond to the following formulae: 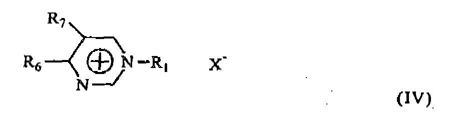 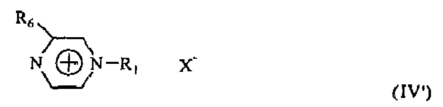
in which R₁ represents an alkyl group comprising from 1 to 18 carbon atoms, preferably from 1 to 12 carbon atoms,
R₆ and R₇, which are identical or different, each represent a hydrogen atom or an alkyl group comprising from 1 to 5 carbon atoms and preferably from 1 to 4 carbon atoms, and
X⁻ represents an anion.

7. Method according to Claim 1, **characterized in that** the tetraalkylphosphonium salts correspond to the following formula: 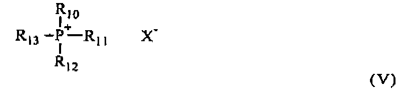
in which R₁₀, R₁₁, R₁₂ and R₁₃, which are identical or different, represent an alkyl group comprising from 1 to 18 carbon atoms and preferably from 1 to 14 carbon atoms, and
X⁻ represents an anion.

8. Cosmetic method according to any one of the preceding claims, **characterized in that** the anions are chosen from chloride (Cl⁻), bromide (Br⁻), tetrachloroaluminate (AlCl₄⁻), tetrachloronickel (NiCl₄⁻), perchlorate (ClO₄⁻), nitrate (NO₃⁻), nitrite (NO₂⁻), sulphate (SO₄²⁻), methyl sulphate (CH₃SO₄⁻), tetrafluoroborate (BF₄⁻), hexafluorophosphate (PF₆⁻), hexafluoroantimonate (SbF₆⁻), triflate [TfO] (CF₃SO₂⁻), nonaflate [NfO] (CF₃(CF₂)₃SO₂⁻), bis (triflyl) amide [Tf₂N] ((CF₃SO₂)₂N⁻), trifluoroacetate [TA] (CF₃CO₂⁻), heptafluorobutanoate [HB] (CF₃(CF₂)₃CO₂⁻), acetate (CH₃CO₂⁻), trifluoroacetate (CF₃CO₂⁻) and trifluoromethanesulphonate (CF₃SO₃⁻) ions.

9. Cosmetic method according to Claim 1, **characterized in that** the organic salt is chosen from:
1-ethyl-3-methylimidazolium bromide,
1-butyl-3-methylimidazolium chloride,
1-hexyl-3-methylimidazolium chloride,
1-methyl-3-octylimidazolium chloride,
1-decyl-3-methylimidazolium chloride,
1-decyl-3-methylimidazolium bromide,
1-dodecyl-3-methylimidazolium chloride,
1-methyl-3-tetradecylimidazolium chloride,
4-methyl-N-butylpyridinium chloride,
3-methyl-N-butylpyridinium chloride,
4-methyl-N-hexylpyridinium chloride,
1-ethyl-3-methylimidazolium tetrafluoroborate,
1-butyl-3-methylimidazolium tetrafluoroborate,
1-pentyl-3-methylimidazolium tetrafluoroborate,
1-hexyl-3-methylimidazolium tetrafluoroborate,
1-heptyl-3-methylimidazolium tetrafluoroborate,
1-octyl-3-methylimidazolium tetrafluoroborate,
1-nonyl-3-methylimidazolium tetrafluoroborate,
1-decyl-3-methylimidazolium tetrafluoroborate,
4-methyl-N-butylpyridinium tetrafluoroborate,
1-hexyl-3-ethylimidazolium tetrafluoroborate,
1-ethyl-3-methylimidazolium hexafluorophosphate,
1-butyl-3-methylimidazolium hexafluorophosphate,
1-pentyl-3-methylimidazolium hexafluorophosphate,
1-hexyl-3-methylimidazolium hexafluorophosphate,
1-heptyl-3-methylimidazolium hexafluorophosphate,
1-octyl-3-methylimidazolium hexafluorophosphate,
1-nonyl-3-methylimidazolium hexafluorophosphate,
1-decyl-3-methylimidazolium hexafluorophosphate,
1,3-dimethylimidazolium methyl sulphate,
1-methyl-3-butylimidazolium methyl sulphate,
1-ethyl-3-methylimidazolium nitrate,
1-ethyl-3-methylimidazolium nitrite,
1-ethyl-3-methylimidazolium acetate,
1-ethyl-3-methylimidazolium sulphate,
1-ethyl-3-methylimidazolium triflates,
1-ethyl-3-methylimidazolium nonaflates,
1-ethyl-3-methylimidazolium bis(triflyl)amide,
1-butylpyridinium bromide,
1-butylpyrimidinium trifluoromethanesulphonate,
1-hexylpyrimidinium trifluoromethanesulphonate,
trihexyltetradecylphosphonium chloride,
tributyltetradecylphosphonium chloride,
1-ethyl-2-methylpyrazolium tetrafluoroborate,
1-methyl-3-butylpyrimidinium tetrafluoroborate, and
1-ethyl-3-methylimidazolium trifluoroacetate.

10. Cosmetic method according to any one of the preceding claims, **characterized in that** the organic salt is present in an amount of between 0.00001% and 99.9999%, preferably between 0.01% and 50%, by weight, with respect to the total weight of the composition.

11. Cosmetic method according to any one of the preceding claims, **characterized in that** the cosmetically acceptable medium comprises a cosmetically acceptable compound or a mixture of cosmetically acceptable compounds which makes it possible to convey the organic salts.

12. Method according to Claim 11, **characterized in that** the cosmetically acceptable medium is chosen from water; C₁-C₄ aliphatic alcohols; aromatic alcohols; fatty alcohols; modified or unmodified polyols; volatile or nonvolatile silicones; mineral, organic or vegetable oils; waxes which are or are not oxyethylenated, paraffins; C₅-C₁₀ alkanes; fatty acids; fatty amides or fatty esters.

13. Cosmetic method according to any one of the preceding claims, **characterized in that** the composition also comprises at least one organic compound chosen from acetone, methyl ethyl ketone, methyl acetate, butyl acetate, ethyl acetate, dimethoxyethane and diethoxyethane.

14. Cosmetic method according to any one of the preceding claims, **characterized in that** the composition additionally comprises at least one cosmetic additive chosen from reducing agents, oxidizing agents, fatty substances, silicones, thickening agents, softening agents, antifoaming agents, moisturizing agents, emollients, basifying agents, plasticizers, sunscreens, direct or oxidation dyes, pigments, inorganic fillers, clays, colloidal minerals, pearlescent agents, fragrances, peptizing agents, preservatives, anionic, amphoteric, zwitterionic or nonionic surfactants, fixing or nonfixing polymers, conditioning polymers, proteins and vitamins.

15. Use of an organic salt as defined in any one of Claims 1 to 9 as agent for conditioning keratinous substances and in particular the hair.

16. Use of the composition according to any one of Claims 1 to 14 for washing and/or conditioning keratinous substances and in particular the hair.

## Patentansprüche

1. Verfahren zur kosmetischen Behandlung von Keratinsubstanzen und insbesondere Haaren, das darin besteht, auf die Keratinsubstanzen bei einer Temperatur von 10 bis 80 °C eine kosmetische Zusammensetzung aufzubringen, die in einem kosmetisch akzeptablen Medium mindestens ein organisches Salz enthält, das einen Schmelzpunkt unter 60 °C aufweist, und gegebenenfalls nach einer Einwirkzeit von 15 Sekunden bis 30 Minuten zu spülen, wobei das organische Salz unter den Salzen von Imidazolium, Pyrazolium, Pyridinium, Pyrimidinium und Tetraalkylphosphonium und deren Gemischen ausgewählt ist.

2. Verfahren nach Anspruch 1, **dadurch gekennzeichnet, dass** die Imidazoliumsalze der folgenden Formel entsprechen: 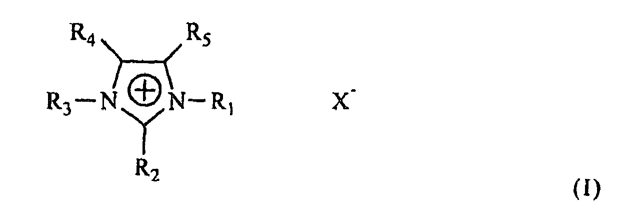
worin die Gruppen R₁ und R₃, die gleich oder verschieden sind, jeweils eine Alkylgruppe mit 1 bis 12 Kohlenstoffatomen bedeuten,
die Gruppen R₂, R₄ und R₅, die gleich oder verschieden sind, jeweils ein Wasserstoffatom oder eine Alkylgruppe mit 1 bis 4 Kohlenstoffatomen bedeuten, und
X⁻ ein Anion ist.

3. Verfahren nach Anspruch 2, **dadurch gekennzeichnet, dass** die Gruppen R₁ und R₃, die gleich oder verschieden sind, jeweils eine Alkylgruppe mit 1 bis 5 Kohlenstoffatomen bedeuten.

4. Verfahren nach Anspruch 1, **dadurch gekennzeichnet, dass** die Pyrazoliumsalze der folgenden Formel entsprechen: 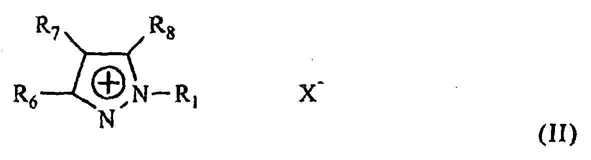
worin R₁ eine Alkylgruppe mit 1 bis 18 Kohlenstoffatomen und vorzugsweise 1 bis 12 Kohlenstoffatomen bedeutet,
die Gruppen R₆, R₇ und R₈, die gleich oder verschieden sind, jeweils ein Wasserstoffatom oder eine Alkylgruppe mit 1 bis 5 Kohlenstoffatomen und vorzugsweise 1 bis 4 Kohlenstoffatomen bedeuten, und
X⁻ ein Anion ist.

5. Verfahren nach Anspruch 1, **dadurch gekennzeichnet, dass** die Pyridiniumsalze der folgenden Formel entsprechen: 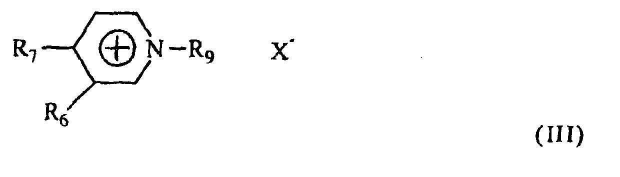
worin R₉ eine Alkylgruppe mit 1 bis 8 Kohlenstoffatomen und vorzugsweise 1 bis 4 Kohlenstoffatomen bedeutet,
die Gruppen R₆ und R₇, die gleich oder verschieden sind, jeweils ein Wasserstoffatom oder eine Alkylgruppe mit 1 bis 5 Kohlenstoffatomen und vorzugsweise 1 bis 4 Kohlenstoffatomen bedeuten, und
X⁻ ein Anion ist.

6. Verfahren nach Anspruch 1, **dadurch gekennzeichnet, dass** die Pyrimidiniumsalze den folgenden Formeln entsprechen: 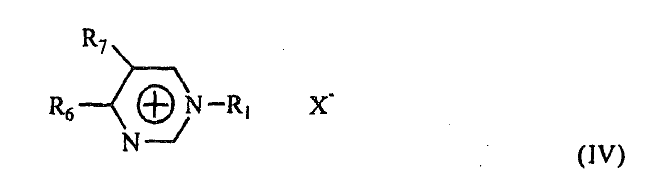 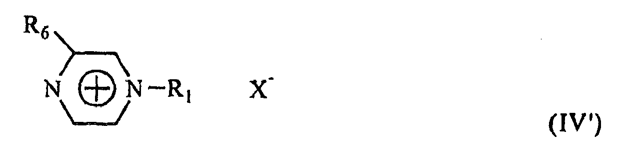
worin R₁ eine Alkylgruppe mit 1 bis 18 Kohlenstoffatomen und vorzugsweise 1 bis 12 Kohlenstoffatomen bedeutet,
die Gruppen R₆ und R₇, die gleich oder verschieden sind, jeweils ein Wasserstoffatom oder eine Alkylgruppe mit 1 bis 5 Kohlenstoffatomen und vorzugsweise 1 bis 4 Kohlenstoffatomen bedeuten, und
X⁻ ein Anion ist.

7. Verfahren nach Anspruch 1, **dadurch gekennzeichnet, dass** die Tetraalkylphosphoniumsalze der folgenden Formel entsprechen: 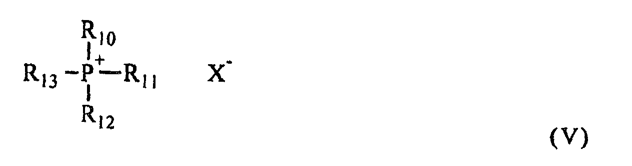
worin R₁₀, R₁₁, R₁₂ und R₁₃, die gleich oder verschieden sind, eine Alkylgruppe mit 1 bis 18 Kohlenstoffatomen und vorzugsweise 1 bis 14 Kohlenstoffatomen bedeuten, und
X⁻ ein Anion ist.

8. Kosmetisches Verfahren nach einem der vorhergehenden Ansprüche, **dadurch gekennzeichnet, dass** die Anionen ausgewählt sind unter den Ionen Chlorid (Cl⁻), Bromid (Br⁻), Tetrachloraluminat (AlCl₄⁻), Tetrachlornickel (NiCl₄⁻), Perchlorat (ClO₄⁻), Nitrat (NO₃⁻), Nitrit (NO₂⁻), Sulfat (SO₄²⁻), Methylsulfat (CH₃SO₄⁻), Tetrafluorborat (BF₄-), Hexafluorphosphat (PF₆⁻), Hexafluorantimonat (SbF₆⁻), Triflat [TfO] (CF₃SO₂⁻), Nonaflat [NfO] (CF₃(CF₂)₃SO₂⁻), Bis(trifyl)amid [Tf₂N] (CF₃SO₂)₂N⁻), Trifluoracetat [TA] (CF₃CO₂⁻), Heptafluorbutanoat [HB] (CF₃(CF₂)₃CO₂⁻), Acetat (CH₃CO₂⁻), Trifluoracetat (CF₃CO₂⁻) und Trifluormethansulfonat (CF₃SO₃⁻).

9. Kosmetisches Verfahren nach Anspruch 1, **dadurch gekennzeichnet, dass** das organische Salz ausgewählt ist unter:
1-Ethyl-3-methylimidazoliumbromid,
1-Butyl-3-methylimidazoliumchlorid,
1-Hexyl-3-methylimidazoliumchlorid,
1-Methyl-3-octylimidazoliumchlorid,
1-Decyl-3-methylimidazoliumchlorid,
1-Decyl-3-methylimidazoliumbromid,
1-Dodecyl-3-methylimidazoliumchlorid,
1-Methyl-3-tetradecylimidazoliumchlorid,
4-Methyl-N-butylpyridiniumchlorid,
3-Methyl-N-butylpyridiniumchlorid,
4-Methyl-N-hexylpyridiniumchlorid,
1-Ethyl-3-methylimidazolium-tetrafluorborat,
1-Butyl-3-methylimidazolium-tetrafluorborat,
1-Pentyl-3-methylimidazolium-tetrafluorborat,
1-Hexyl-3-methylimidazolium-tetrafluorborat,
1-Heptyl-3-methylimidazolium-tetrafluorborat,
1-Octyl-3-methylimidazolium-tetrafluorborat,
1-Nonyl-3-methylimidazolium-tetrafluorborat,
1-Decyl-3-methylimidazolium-tetrafluorborat,
4-Methyl-N-butylpyridinium-tetrafluorborat,
1-Hexyl-3-ethylimidazolium-tetrafluorborat,
1-Ethyl-3-methylimidazolium-hexafluorphosphat,
1-Butyl-3-methylimidazolium-hexafluorphosphat,
1-Pentyl-3-methylimidazolium-hexafluorphosphat,
1-Hexyl-3-methylimidazolium-hexafluorphosphat,
1-Heptyl-3-methylimidazolium-hexafluorphosphat,
1-Octyl-3-methylimidazolium-hexafluorphosphat,
1-Nonyl-3-methylimidazolium-hexafluorphosphat,
1-Decyl-3-methylimidazolium-hexafluorphosphat,
1,3-Dimethylimidazolium-methylsulfat,
1-Methyl-3-butylimidazolium-methylsulfat,
1-Ethyl-3-methylimidazoliumnitrat,
1-Ethyl-3-methylimidazoliumnitrit,
1-Ethyl-3-methylimidazoliumacetat,
1-Ethyl-3-methylimidazoliumsulfat,
1-Ethyl-3-methylimidazoliumtriflate,
1- Ethyl-3-methylimidazoliumnonaflate,
1-Ethyl-3-methylimidazolium-bis(trifyl)amid,
1-Butylpyridiniumbromid,
1-Butylpyrimidinium-trifluormethansulfonat,
1-Hexylpyrimidinium-trifluormethansulfonat,
Trihexyl-tetradecylphosphoniumchlorid,
Tributyl-tetradecylphosphoniumchlorid,
1-Ethyl-2-methylpyrazolium-tetrafluorborat,
1-Methyl-3-butylpyrimidinium-tetrafluorborat, und
1-Ethyl-3-methylimidazolium-trifluoracetat.

10. Kosmetisches Verfahren nach einem der vorhergehenden Ansprüche, **dadurch gekennzeichnet, dass** das organische Salz in einer Menge von 0,00001 bis 99,9999 % und vorzugsweise 0,01 bis 50 Gew.-%, bezogen auf das Gesamtgewicht der Zusammensetzung, enthalten ist.

11. Kosmetisches Verfahren nach einem der vorhergehenden Ansprüche, **dadurch gekennzeichnet, dass** das kosmetisch akzeptable Medium eine kosmetisch akzeptable Verbindung oder ein Gemisch von kosmetisch akzeptablen Verbindungen enthält, die als Träger für die organischen Salze dienen können.

12. Verfahren nach Anspruch 11, **dadurch gekennzeichnet, dass** das kosmetisch akzeptable Medium unter Wasser; aliphatischen C₁₋₄-Alkoholen; aromatischen Alkoholen; Fettalkoholen; modifizierten oder nichtmodifizierten Polyolen; flüchtigen oder nichtflüchtigen Siliconen; anorganischen, organischen oder pflanzlichen Ölen; ethoxylierten oder nicht ethoxylierten Wachsen, Paraffinen; C₅₋₁₀-Alkanen; Fettsäuren; Fettamiden, Fettsäureestern ausgewählt ist.

13. Kosmetisches Verfahren nach einem der vorhergehenden Ansprüche, **dadurch gekennzeichnet, dass** die Zusammensetzung ferner mindestens eine organische Verbindung enthält, die unter Aceton, Methylethylketon, Methylacetat, Butylacetat, Ethylacetat, Dimethoxyethan und Diethoxyethan ausgewählt ist.

14. Kosmetisches Verfahren nach einem der vorhergehenden Ansprüche, **dadurch gekennzeichnet, dass** die Zusammensetzung ferner mindestens einen kosmetischen Zusatzstoff enthält, der unter den Reduktionsmitteln, Oxidationsmitteln, Fettsubstanzen, Siliconen, Verdickungsmitteln, beruhigenden Stoffen, Schaumverhütungsmitteln, Hydratisierungsmitteln, Emollientien, Alkalisierungsmitteln, Weichmachern, Sonnenschutzfiltern, Direktfarbstoffen und oxidativen Farbstoffen, Pigmenten, mineralischen Füllstoffen, Tonen, kolloidalen Mineralien, Perlglanzstoffen, Parfums, Peptisierungsmitteln, Konservierungsmitteln, anionischen, amphoteren, zwitterionischen oder nichtionischen grenzflächenaktiven Stoffen, fixierenden oder nicht fixierenden Polymeren, konditionierenden Polymeren, Proteinen und Vitaminen ausgewählt ist.

15. Verwendung eines organischen Salzes, wie es in einem der Ansprüche 1 bis 9 definiert ist, als Konditioniermittel für Keratinsubstanzen und insbesondere Haare.

16. Verwendung der Zusammensetzung nach einem der Ansprüche 1 bis 14 für die Reinigung und/oder Konditionierung von Keratinsubstanzen und insbesondere Haaren.
